(19) Europäisches Patentamt
European Patent Office
Office européen des brevets

(11) Numéro de publication : **0 587 471 A1**

(12) **DEMANDE DE BREVET EUROPEEN**

(21) Numéro de dépôt : **93402052.0**

(22) Date de dépôt : **13.08.93**

(51) Int. Cl.[5] : **C07B 37/12,** C07B 41/02, C07B 53/00, C07H 15/10, C07C 29/143

(30) Priorité : **28.08.92 FR 9210346**

(43) Date de publication de la demande : **16.03.94 Bulletin 94/11**

(84) Etats contractants désignés : **AT BE CH DE DK ES FR GB GR IE IT LI NL PT SE**

(71) Demandeur : **ERIDANIA BEGHIN-SAY**
**F-59239 Thumeries (FR)**

(72) Inventeur : **Lubineau, André**
**2, allée des Prés, Hameau de Rouillon**
**F-91410 Dourdan (FR)**
Inventeur : **Scherrmann, Marie-Christine**
**7A, rue Basse**
**F-57180 Terville (FR)**
Inventeur : **Mentech, Julio**
**1D, rue Phélypeaux**
**F-69100 Villeurbanne (FR)**

(74) Mandataire : **David, Daniel et al**
**KAYSERBERG Service Propriété Industrielle**
**23 boulevard Georges Clemenceau**
**F-92402 Courbevoie Cédex (FR)**

(54) **Utilisation d'hydrates de carbone à l'état solide comme support chiral de réactions chimiques.**

(57) L'invention concerne une nouvelle utilisation des mono-, di- et trisaccharides et de leurs alkylglucosides dont le groupement alkyle a de 1 à 4 atomes de carbone. à l'état solide. comme support chiral de réactions chimiques.

Elle concerne également des procédés chimiques mettant en oeuvre un support solide constitué de mono-, di- ou trisaccharides ou de leurs alkylglucosides dont le groupement alkyle a de 1 à 4 atomes de carbone.

EP 0 587 471 A1

La présente invention concerne l'utilisation d'hydrates de carbone à l'état solide comme support chiral de réactions chimiques.

Il existe actuellement un besoin industriel général de pouvoir modifier la sélectivité de réactions organiques, notamment la régiosélectivité, la stéréosélectivité, l'énantiosélectivité et la chimiosélectivité. Diverses solutions ont été proposées à cet égard, en fonction du problème particulier à résoudre, parmi lesquelles on peut citer l'utilisation de solvants chiraux mais le coût de ces derniers est si élevé que des procédés mettant en jeu des réactions effectuées dans de tels solvants sont peu intéressants du point de vue économique.

La demanderesse à décrit dans la demande de brevet PCT WO 90/12773 un milieu réactionnel pouvant notamment permettre d'augmenter sensiblement la vitesse de réactions organiques et de modifier le cas échéant leur sélectivité tout en paliant les difficultés énoncées ci-dessus. Ce milieu réactionnel pour des réactions organiques consiste en une solution aqueuse concentrée d'au moins un hydrate de carbone choisi parmi les mono-, di- et trisaccharides et leurs alkyl-glycosides dont le groupement alkyle a de 1 à 4 atomes de carbone.

On assiste actuellement au développement d'une nouvelle catégorie de réactions chimiques dites réactions chimiques supportées dans lesquelles la présence d'un support solide favorise ladite réaction. D'une façon générale, les supports solides utilisés sont des produits de type argile, silice, alumine, zéolite, montmorillonite. Il ne s'agit donc nullement de supports chiraux.

A titre d'exemples de document général concernant la chimie supportée, on citera l'ouvrage intitulé "Preparation chemistry using supported reagents" édité par P. LAZLO, Academic Press, Londres, 1987.

Différentes études ont porté sur des réactions énantiosélectives ou diastéréosélectives réalisées en présence d'un support solide.

A titre d'exemples de tels travaux on citera "cyclo-addition reactions of dimethyl maleate and fumarate with cyclo-pentadiene on $\gamma$-alumina. Competing mechanisms for epimerization of the DIELS-ADLER adducts" S. BAINS et al, Tetrahedron Letters, Vol 32, N° 41, pp 5563-5666, 1991.

On citera également l'utilisation de $\beta$-cyclodextrine comme complexe d'inclusion pour la réduction énantiosélective des cétones décrites dans Carbohydrate Research, 192, (1989), pp 363-365.

La demanderesse a maintenant découvert que l'utilisation de mono-, di- et trisaccharides et de leurs alkylglucosides dont le groupement alkyle a de 1 à 4 atomes de carbone, à l'état solide, comme support de réactions chimiques permettait de réaliser des réactions stéréosélectives ou énantiosélectives.

Ainsi selon une de ses caractéristiques essentielles, l'invention concerne l'utilisation des mono-, di-, et trisaccharides et de leurs alkyl-glucosides dont le groupement alkyle a de 1 à 4 atomes de carbone, à l'état solide, comme support chiral de réactions chimiques.

A titre d'exemples non limitatifs d'hydrates de carbone utilisables dans la présente invention on peut citer le glucose, le fructose, le saccharose, le ribose, le galactose, le mannose, l'$\alpha$-méthyl glucoside.

Le support selon l'invention sera avantageusement utilisé dans toutes réactions dont l'un au moins des réactifs est susceptible d'interagir, par exemple par une Liaison de type hydrogène avec le support.

Le support selon l'invention est utilisé dans des réactions dont on veut modifier la diastéréosélectivité ou l'énantiosélectivité.

A titre d'exemples non limitatifs le support de l'invention permet d'augmenter la diastéréosélectivité de produits de réaction de type DIELS-ADLER ou de créer la chiralité dans un produit de réaction, par exemple la réduction d'une cétone prochirale en alcool.

Selon un autre aspect de l'invention, elle concerne des procédés chimiques mettant en oeuvre des supports décrits précédemment dans des réactions chimiques dites supportées.

Selon une première variante de ces procédés le mélange du support solide avec le (ou les) réactif(s) solide(s) résulte d'une réaction de coévaporation après mise en solution dans un solvant commun.

Selon une autre variante du procédé, le mélange du support solide et du ou des réactifs à l'état solide est réalisé par lyophilisation d'une solution des deux produits dans un solvant commun.

Selon encore une autre variante de l'invention, le mélange du support solide et du ou des réactifs solides est réalisé par cobroyage fin des deux produits.

Les exemples suivants, sont donnés à titre purement illustratif de la mise en oeuvre des hydrates de carbone à l'état solide comme supports chiraux, de réactions chimiques supportées.

## Exemples

### Exemple 1

Réaction de type Diels-Adler entre le 1-β-D-glucosyl-1,3-butadiène et la méthyl vinyl cétone.

Le 1-β-D-glucosyl-1,3-butadiène (ci-après dénommé produit 1) a été préparé comme indiqué dans la publication de Lubineau A, Queneau Y, J. Org. chem. 1987, 57, 1001.

La réaction entre le diène (1) et la méthyl vinyl cétone (produit 2) se fait selon le schéma réactionnel suivant :

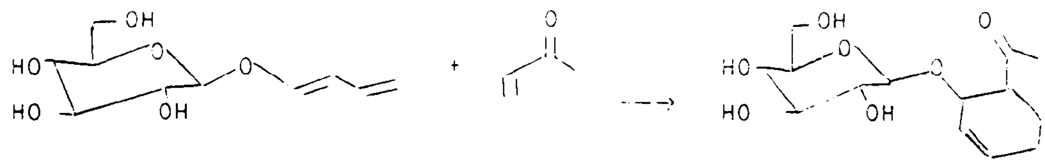

1 2 3

La réaction a été menée à 25° sous pression atmosphérique dans différents solvants ou sans solvant (exemples donnés - titre comparatif) ou sur support solide selon l'invention.

1. a) Réaction dans l'eau puis acétylation. (comparatif)

200 mg du diène (1) (0,862 mmol) et 60,5 mg de méthyl vinyl cétone (8,62 mmol) dans 2 ml d'eau sont agités une nuit à température ambiante.
Après évaporation de l'eau, le mélange brut est acétylé (anhydride acétique, pyridine.)
La flash chromatographie (éluant : acétate d'éthyle, hexane 1:1) permet d'isoler 300 mg de produit (74 %) qui est caractérisé par RMN $^1$H.

1. b) Réaction dans une solution de glucose 4 M. (comparatif)

Le protocole suivi est le même que dans l'eau. Les adduits formés sont séparés du glucose par extraction continue à l'acétate d'éthyle (75 %) puis flash chromatographie (acétate d'éthyle/méthanol 4:1).

1. c) Réaction dans une solution de saccharose 2 M. (Comparatif)

Le protocole suivi est le même que dans l'eau. Les adduits formés sont séparés du saccharose par extraction continue à l'acétate d'éthyle.

1. d) Réaction sans solvant : (comparatif)

300 mg de diène (1) (1,29 mmol) et 0,9 g (12,9 mmol) de méthyl vinyl cétone sont agités une nuit à température ambiante dans un ballon soigneusement bouché. La méthyl vinyl cétone en excès est évaporée (évaporateur rotatif). On obtient 354 mg de produit (90 %) après flash chromatographie (acétate d'éthyle, méthanol 4:1).

1. e) Réaction sur sucre solide (selon l'invention)

On à réalisé trois modes différents de dépot du diène sur le sucre.

- coévaporation :

  150 mg de diène (1) (0,64 mmol) et 400 mg de saccharose sont dissous dans 10 ml d'eau. La solution est évaporée puis le résidu est repris deux fois dans 10 ml d'eau que l'on évapore. Les parois sont rincées avec un minimum d'éthanol et on évapore à nouveau. Le ballon est placé au dessiccateur sur $P_2O_5$, KOH et $CaCl_2$ pendant une nuit.
- lyophilisation :

  150 mg de diène (1) (0,64 mmol) et 400 mg de saccharose sont dissous dans 20 ml d'eau. Le mélange est lyophilisé (une nuit).

- broyage :

  150 mg de diène (1) (0,64 mmol) et 400 mg de saccharose sont broyés finement puis placés dans un ballon.

Aux échantillons préparés comme décrit ci-dessus, on ajoute en très fines gouttes 224 mg de méthyl vinyl cétone (3,2 mmol).

Le flacon est bouché soigneusement.

Après une soixantaine d'heures à température ambiante, on évapore la méthyl vinyl cétone en excès. En général les adduits n'ont pas été isolés (injection du mélange brut en HPLC). On peut cependant les séparer du sucre par extraction.

Dans les différents exemples ci-dessus, on a déterminé les proportions entre les adduits obtenus par HPLC analytique ou par RMN$^{13}$C du mélange brut. Dans ce cas, on dose par intégration des carbones anomères dont les temps de relaxation peuvent êtres estimés semblables, car l'environnement chimique de ceux-ci dans les adduits est très voisin. La hauteur respective de ces signaux donne alors une bonne représentation de leurs proportions.

On a vérifié que les pourcentages donnés par l'une ou l'autre méthode étaient les mêmes.

Le mélange des adduits a néanmoins été isolé (sous forme libre ou acétylée) afin d'en vérifier la structure.

La RMN $^1$H des adduits acétylés montre que les diastéréoisomères endo sont obtenus très majoritairement (couplage cis entre les protons 1,2 du cycle formé.)

L'attribution de la sélectivité faciale dans l'état de transition endo résulte de la comparaison des spectres RMN de nos produits avec les adduits suivants dont la configuration absolue a été déterminée grâce aux données spectrals et optiques.

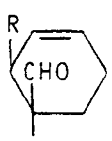
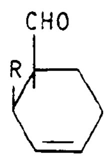
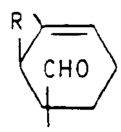
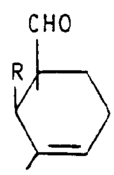
avec
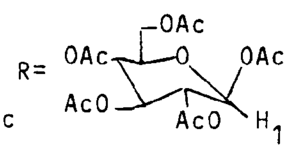

6a 6b 7a 7b

Après acétyLation, les produits que nous obtenons sont :

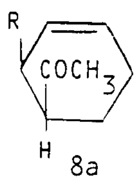
8a

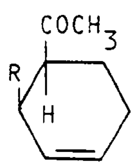
8b

| | pics $H_1$ | |
|---|---|---|
| 6 a,b | 4,68 (minoritaire) | 4,63 (majoritaire) |
| 7 a,b | 4,68 (minoritaire) | 4,59 (majoritaire) |
| 8 a,b | 4,68 (minoritaire) | 4,63 (majoritaire). |

On se reportera pour ces données aux publications dans Tetrahedron Letters, 1985, 26, 2953, J.Org. Chem. 1987, 52, 1001 Tetrahedron Letters, 1989, 45, 6697.

Le spectre RMN du proton de 8a et b montre comme dans le cas de 6 et 7, un déblindage plus faible du proton anomère de l'unité glucose et une plus faible séparation des protons oléfiniques pour l'isomère majoritaire. La configuration endo Re ayant été attribuée aux isomères majoritaires 6a et 7a nous pouvons déduire que l'isomère majoritaire est 8a de configuration endo Re.

Les résultats obtenus sont rassemblés dans le tableau 1.

Tableau 1 : résultats de la réaction entre le diène 1 et la méthyl vinyl cétone dans différentes conditions.

| Solvant | Mode de dépôt du diène sur le sucre | Endo Re/Endo Si |
|---|---|---|
| $H_2O$ | - | 60/40 |

Tableau 1 (suite)

| Solvant | Mode de dépôt du diène sur le sucre | Endo Re/Endo Si |
|---|---|---|
| glucose 4M | - | 60/40 |
| saccharose 2M | - | 60/40 |
| - | - | 70/30 |
| saccharose solide | coévaporation | 88/12 |
| | lyophilisation | 78/22 |
| | broyage | 76/24 |

Les concentrations en diène par rapport au "sucre solvant" utilisées sont de 0,3 mg/mg de glucose et 0,375 mg/mg de saccharose. Si on diminue la quantité de sucre jusqu'à 1 mg/mg de sucre, on retrouve le pourcentage de 70/30 obtenu sans solvant.

D'une manière générale, il ressort que les sucres en solution (glucose ou saccharose) n'influent pas le déroulement stéréochimique de la réaction puisque l'on retrouve les mêmes résultats que dans l'eau (60/40).

La meilleure sélectivité a été obtenue sur saccharose solide laissant supposer que la matrice diène-saccharose est plus structurée que dans les autres cas.

Avec le saccharose, on remarque également que le mode de dépôt influence la sélectivité de la réaction.

Exemple 2 : Réduction de l'acétophénone par $NaBH_4$

On a réalisé cette réduction à titre comparatif dans différents solvants dans les conditions indiquées en (a) ci-dessous et sur support solide dans les conditions indiquées en (b) ci-dessous.

a) en solution (comparatif).

A une émulsion de 500 mg (4,1 mmol) d'acétophénone dans 20 ml de solvant (eau, saccharose 2M, ou α-méthyl glucoside 2,5M), on ajoute 1,2 équivalents de $NaBH_4$ (1,7 équivalents dans les solutions de sucre.)

La réaction est suivie par chromatographie en couche mince.

Après neutralisation par de l'acide acétique 10 %, l'alcool est extrait à l'éther et purifié par flash chromatographie (acétate d'éthyle/hexane 1:9).

b) en phase solide (selon l'invention).

Le sucre et la cétone ou le sucre et le borohydure de sodium sont mélangés par broyage dans un mortier. On ajoute ensuite le borohydure de sodium ou la cétone. Le milieu réactionnel est broyé puis placé dans un flacon sec.

La poudre est diluée dans de l'eau, cette solution est neutralisée et extraite. L'alcool est purifié par flash chromatographie comme en phase liquide.

Dans les différents essais, on a déterminé l'excès énantiométrique (ee) par mesure du pouvoir rotatoire du mélange obtenu après extraction et purification par flash chromatographie. La pureté des échantillons a été vérifiée par RMN $^1H$.

Les conditions expérimentales et les résultats sont consignés dans le tableau 2 ci-dessous :

Tableau 2

| Agent de réduction (eq) [a] | Solvant (eq) [b] | Conditions [c] | Rdt [d] | e.e. [e] configuration) |
|---|---|---|---|---|
| $NaBH_4$ (1,2) | $H_2O$ | 40 min TA | 99 % | 0 |
| $NaBH_4$ (10) | αMeglc S (8) | I 70 h TA | 80 % | 2,8(S) |
| $NaBH_4$ (10) | sac S (8) | I 70 h TA | 84 % | 2,4(R) |
| $NaBH_4$ (10) | sac S (15) | I 70 h TA | 84 % | 1,65(R) |
| $NaBH_4$ (10) | αMeglc S (15) | I 70 h TA | 78 % | 1,9(S) |
| $NaBH_4$ (10) | sac S (8) | II 46 h TA | 80 % | 1,27(R) |
| $NaBH_4$ (10) | sac S (10) | II 70 h TA | 75 % | 1,4(R) |
| $NaBH_4$ (10) | αMeglc S (8) | III 46 h TA | 78 % | 2,6(S) |
| $NaBH_4$ (1,7) | sac 2 M | 60 min TA | 80 % | 2,1(S) |
| $NaBH_4$ (1,7) | αMeglc 2,5 M | 60 min TA | 68 % | 2,2(R) |

(a) eq désigne le nombre d'équivalents par rapport à la cétone

(b) §Meglc S désigne l'§-méthyl glucoside solide

sac S désigne le saccharose solide

(eq) désigne le nombre d'équivalents par rapport à la cétone

(c) I broyage du sucre avec $NaBH_4$ puis ajout de la cétone

II dépôt de la cétone sur le sucre par évaporation du solvant

III broyage du sucre et de la cétone avant ajout de $NaBH_4$

TA température ambiante

(d) rendement en produit isolé

(e) e.e. désigne l'excès énantiométrique en pourcentage.

Exemple 3

Réduction de la trans-4-phényl 3 butène-2 one.

a) en solution (comparatif).

1 g (6,8 mmol) de cétone est mis en suspension dans 15 ml de solvant. Le milieu est chauffé jusqu'à ce que la cétone fonde. La suspension est refroidie à température ambiante avec une agitation vigoureuse. Il se forme alors une pâte à laquelle on ajoute 1,2 équivalents de $NaBH_4$.

La réaction est suivie par chromatographie en couche mince.

Après neutralisation par $CH_3CO_2H$ à 10 %, l'alcool est extrait à l'éther et purifié par flash chromatographie (acétate d'éthyle/hexane 1:9).

b) en phase solide. (selon l'invention)

Le protocole est le même que pour la réduction de l'acétophénone de l'exemple 2.

La détermination des excès énantiométriques a été faite par RMN du proton en présence de 15 % de tris (3-heptafluoropropylhydroxyméthylène) d camphorato europium III.

Les conditions et les résultats sont donnés dans le tableau 3 ci-dessous où les abréviations et annotations ont la même signification que dans le tableau 2.

Tableau 3 : réduction de (E) Ph CH = $CHCOCH_3$

| Agent de réduction (eq)[a] | Solvant (eq)[b] | Conditions[c] | Rdt[d] | ee % |
|---|---|---|---|---|
| $NaBH_4$ (1,2) | $H_2O$ | 9 h TA | 79 % | 0 |
| $NaBH_4$ (10) | §Meglc S (20) | III 70 h TA | 72 % | 3 |
| $NaBH_4$ (5) | sac S (100) | III 70 h TA | 60 % | 0 |
| $NaBH_4$ (5) | sac S (100) | I 70 h TA | 63 % | 5 * |

* isomère majoritaire différent.

## Revendications

1. Utilisation des mono-, di- et trisaccharides et de leurs alkylglucosides dont le groupement alkyle a de 1 à 4 atomes de carbone, à l'état solide, comme support chiral de réactions chimiques.
2. Utilisation selon la revendication 1 caractérisée en ce que la réaction chimique est une réaction de type Diels-Adler.
3. Utilisation selon la revendication 1 caractérisée en ce que la réaction chimique est une réduction de cétone prochirale en alcool.
4. Procédé chimique caractérisé en ce qu'il met en oeuvre un support solide constitué de mono-, di- ou trisaccharides ou de leurs alkylglucosides dont le groupement alkyle à de 1 à 4 atomes de carbone.
5. Procédé selon la revendication 4 caractérisé en ce que le support solide est mélangé avec le (ou les) réactif(s) solide(s) par coévaporation après mise en solution dans un solvant commun.
6. Procédé selon la revendication 4, caractérisé en ce que le support solide est mélangé au(x) réactif(s) à l'état solide par lyophilisation d'une solution de ces produits dans un solvant commun.
7. Procédé selon la revendication 4, caractérisé en ce que le support solide et les réactifs à l'état solide sont mélangés par co-broyage.

Office européen des brevets

# RAPPORT DE RECHERCHE EUROPEENNE

Numero de la demande
EP 93 40 2052

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| D,A | WO-A-90 12773 (BEGHIN-SAY)<br>* revendications; exemples *<br>--- | 1-7 | C07B37/12<br>C07B41/02<br>C07B53/00<br>C07H15/10<br>C07C29/143 |
| D,A | CARBOHYDRATE RESEARCH<br>vol. 192 , 23 Octobre 1989 , AMSTERDAM<br>pages 363 - 365<br>F. TODA ET AL 'ENANTIOSELECTIVE REDUCTION OF KETONES IN INCLUSION COMPLEXES WITH CYCLOMALTOHEPTAOSE IN THE SOLID STATE: ACCELERATION OF THE REACTION BY ULTRASOUND'<br>* le document en entier *<br>----- | 1-7 | |

DOMAINES TECHNIQUES RECHERCHES (Int.Cl.5)

C07B
C07H
C07C

Le présent rapport a été établi pour toutes les revendications

| Lieu de la recherche | Date d'achèvement de la recherche | Examinateur |
|---|---|---|
| LA HAYE | 1 Décembre 1993 | Wright, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)